(19) Europäisches Patentamt
European Patent Office
Office européen des brevets

(11) **EP 4 368 577 A1**

(12) **EUROPEAN PATENT APPLICATION**

(43) Date of publication:
**15.05.2024 Bulletin 2024/20**

(21) Application number: **22383073.8**

(22) Date of filing: **08.11.2022**

(51) International Patent Classification (IPC):
*C01G 41/00* (2006.01)    *A61K 33/00* (2006.01)

(52) Cooperative Patent Classification (CPC):
**C01G 41/00; A61K 33/00;** C01P 2002/82;
C01P 2006/80; C01P 2006/82

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **OXOLIFE, S.L.**
**08192 Sant Quirze del Vallès (ES)**

(72) Inventors:
• **ARBAT BUGIÉ, Agnès**
**08192 Sant Quirze Del Valles (ES)**

• **TORRES FERRAZ, Rosa**
**08192 Sant Quirze Del Valles (ES)**
• **BESSA BELLMUNT, Jordi**
**08193 Bellaterra (Barcelona) (ES)**
• **TAMAYO HARO, Abel**
**08193 Bellaterra (Barcelona) (ES)**
• **CANALS ALMAZÁN, Ignacio**
**08192 Sant Quirze Del Valles (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **PROCESS FOR THE PREPARATION OF TUNGSTEN (VI) SALT HYDRATES**

(57)    The present invention provides a process for preparing tungsten (VI) salts hydrates, the process comprising (a) Preparing a tungsten (VI) salt in an alkaline aqueous solution; (b) Filtering the aqueous solution at a temperature at which the tungsten (VI) salt remains dissolved; (c) Precipitating the tungsten (VI) salt hydrate from the filtrate resulting from step (b); (d) Filtering; (e) Washing the resulting filter cake with a water-miscible solvent; and (f) Drying the washed cake under conditions that allow the removal of the water-miscible solvent.

The resulting salts show a level of impurities and solvents so low that they are suitable in the fields of pharmacy, veterinary and dietary.

EP 4 368 577 A1

**Description**

**Field of the invention**

The present invention relates to the chemistry field, particularly to the synthesis of hydrate compounds. The present invention, specifically, refers to a process for preparing tungsten salt hydrates.

**Background of the invention**

It is known that the alkaline (in particular sodium) or ammonium-tungstate or molybdate solutions which form during the alkalization of tungsten and / or molybdenum-containing substances are contaminated with various hydrolyzing ions (silicon, phosphorus, arsenic, etc.). The solution must be cleaned before further processing. According to the known cleaning method, the alkaline solution (pH 13), for example $Na_2WO_4$ solution, is acidified with some acid (for example $H_2SO_4$, HCl) (for example to pH 9) and then an aluminum ion is added; the resulting Al $(OH)_3$ precipitate with a large area binds the impurities in the solution.

The precipitate can be removed by filtration. W-acid is then generated from the remaining pure solution by any method. Tungsten acid can advantageously be produced from the cleaned alkaline or ammonium tungstate solution by cation exchange electrodialysis, and the digestion solution can also be recovered.

This known method has several disadvantageous properties; on the one hand, there are disadvantages of an economic nature, since a pure acid is required for acidification, which increases the cost of the process, and on the other hand, there are further sources of contamination because sulphate or chlorine ions get into the solution.

Therefore, in spite of the efforts made, there is the need of providing processes for the obtaining of tungsten salts complying with quality standards according to the Good Manufacturing Practices (GMP) in the fields of pharmacy, veterinary and dietary supplements.

**Summary of the invention**

The aim of the present invention is to develop a process which is efficient in eliminating the impurities incorporated in the starting tungsten-containing compound, which allows the obtaining of an active pharmaceutical ingredient (API) complying with GMP standards.

When developing the process, however, the inventors identified a further problem when obtaining tungstate metal salts in the form of hydrate: the molecules of water coordinating to the metal salt were extremely labile. As it is shown below, it was found that about a 94% of the starting $Na_2WO_4 \cdot 2H_2O$ lost the molecules of water when vacuum dried at a mild temperature of about 50°C.

In view of the above, therefore, the problem to be solved by the present invention was not only the provision of a process which allowed the obtaining of a tungstate salt hydrate suitable for use in any of above-referred fields of pharma, veterinary or dietary, but that the process guaranteed that the final active product retained the molecules of water at the end of the process.

The present invention solves the above-identified problems with the process of the invention, which comprises a process for obtaining a tungsten (VI) salt hydrate, the process comprising:

(a) Preparing a tungsten (VI) salt in an alkaline aqueous solution;

(b) Filtering the aqueous solution at a temperature at which the tungsten (VI) salt remains dissolved;

(c) Precipitating the tungsten (VI) salt hydrate from the filtrate resulting from step (b);

(d) Filtering;

(e) Washing the resulting filter cake with a water-miscible solvent; and

(f) Drying the washed cake under conditions that allow the removal of the water-miscible solvent.

Steps (a) and (b) guarantees the reduction of the impurities within GMP quality standards being suitable for its use in field of interest related to humans and non-human animals.

Steps (d) and (e) guarantees that the end product, once dried, is stable and retains the molecules of water.

The process of the invention allows the obtaining of a sodium tungstate dihydrate with a remarkable low content of water and residual water-miscible solvent.

In fact, the salt resulting from the process shows a remarkable stability in storage.

Thus, in a second aspect the present invention provides a sodium tungstate dihydrate which is obtainable by the process of the first aspect of the invention.

The resulting sodium tungstate salt dihydrate of the invention is characterized by a water content below 15% w/w with respect the total weight of the salt, and with a level of impurities and residual solvents within the safe levels to make it suitable in the fields of medicine and diet.

Thus, the present invention provides in a third aspect a pharmaceutical, veterinary or dietary sodium tungstate dihydrate comprising a water residual content of up to 15% by weight with respect to the total weight of the salt, particularly a content from 10 to 15% by weight.

In a fourth aspect the present invention provides a pharmaceutical, veterinary or dietary composition comprising a therapeutically effective amount of the sodium tungstate dihydrate of the second and third aspect together with one or more pharmaceutically, veterinary ore dietary acceptable excipients.

In a fourth aspect the present invention provides the sodium tungstate dihydrate as defined in the second or third aspect for use in therapy.

The sodium tungstate dihydrate compound has already been reported in the treatment of female infertility, such as in WO2016012632 or Ballester, 2007 (cf. J. Ballester et. al., "Tungstate administration improves the sexual and reproductive function in female rats with streptozotocin-induced diabetes", Human Reproduction, 2007, vol. 22, pp. 2128-2135). The dihydrate compound of the invention, with such so low levels in water and solvent, would also be suitable in the particular treatment of female infertility.

Therefore, in a fifth aspect the present invention provides the sodium tungstate dihydrate as defined in the second or third aspect for use in the treatment of female infertility, in particular of non-diabetic female infertility. This aspect can alternatively be formulated as the use of the sodium tungstate dihydrate salt as provided in the invention in the manufacture of a medicament for the treatment of female infertility, particularly of non-diabetic female infertility. This aspect can alternatively be formulated as a method for the treatment of female infertility, in particular of non-diabetic female infertility, the method comprising the administration of a therapeutically effective amount of the sodium tungstate dihydrate salt as provided in the invention.

**Description of Drawings**

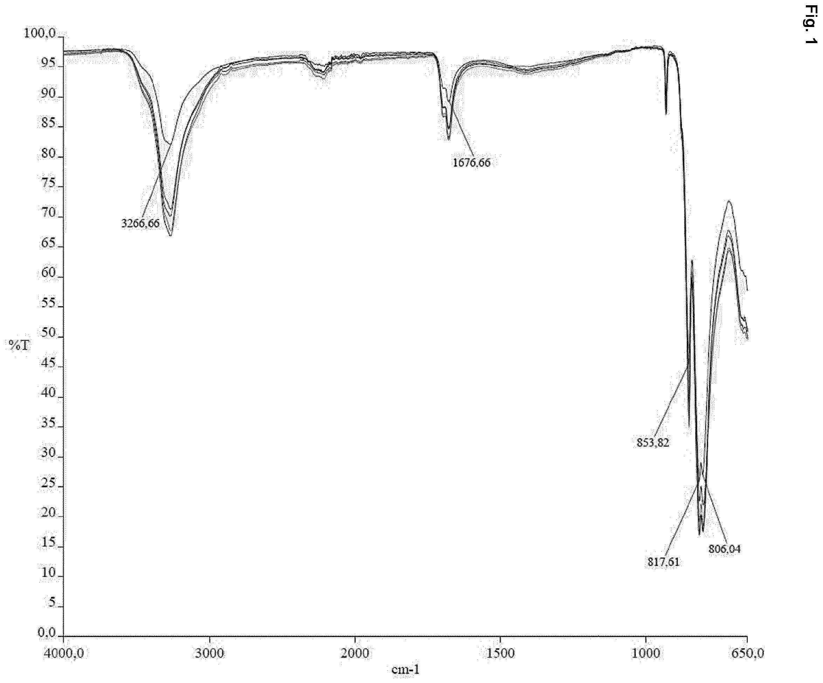

Fig. 1. IR spectrum for $Na_2WO_4 \cdot 2H_2O$ obtained following the process of the invention, starting from two different batches of APT and $WO_3$. The IR was identical in all four cases, identifying the two peaks related to the two water molecules (positions 3266 and 1676 cm$^{-1}$).

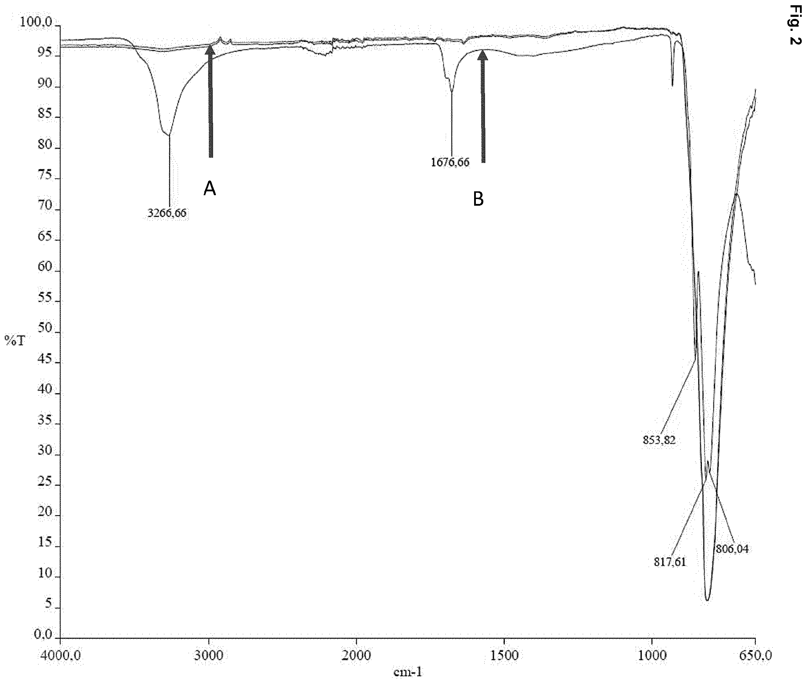

Fig. 2. IRs of the sodium tungstate after drying at 50 and 100 °C (A and B, respectively). IR of the sodium tungstate following the process of the invention. It can be seen that at 50 and 100°C the molecules of water are not present, whereas under the conditions of the process of the invention both molecules are identified (positions 3266 and 1676 cm$^{-1}$).

**Detailed Description**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper end-points of the range.

As used herein, the meaning of the term "comprising" encompasses three alternatives, namely "comprising", "consisting of" and "consisting essentially of".

The present invention provides a process for the preparation of a tungsten (VI) salt hydrate suitable for use in pharma, veterinary and dietary field.

In one embodiment, optionally in combination with any of the embodiments provided above or below, the tungsten (VI) salt hydrate comprises an anion selected from the group consisting of tungstate and isopolytungstate. In another embodiment, optionally in combination with any of the embodiments provided above or below, the anion is tungstate.

In one embodiment, optionally in combination with any of the embodiments provided above or below, the tungsten (VI) salt hydrate comprises a cationic moiety selected from the group of alkaline or alkaline earth metal cations such as: sodium, potassium, magnesium, and calcium; but also of other cations such as ammonium, cupper, zinc and silver. In another embodiment, optionally in combination with any of the embodiments provided above or below, the cation is sodium.

In one embodiment, optionally in combination with any of the embodiments provided above or below, the tungsten (VI) salt hydrate is dihydrate sodium tungstate.

According to the process of the invention, the first step comprises the solubilization of a tungsten-containing compound in an alkaline aqueous solution.

In one embodiment, optionally in combination with any of the embodiments provided above or below, the tungsten-containing compound is selected from: $WO_3$, paratungstate salt, and tungstic acid. Illustrative non-limitative examples of paratungstate salts are ammonium paratungstate (APT), sodium, calcium, or potassium, among others. In one particular embodiment, optionally in combination with any of the embodiments provided above or below, the tungsten-containing compound is $WO_3$.

In the context of the present invention the term "alkaline aqueous solution" can be any of those already known to those skilled in the art. In the context of the present invention, the alkaline solution is any providing a pH value above 7. In one embodiment of the invention, optionally in combination with any of the embodiments provided above or below, the alkaline aqueous solution provides a pH above 8, above 9 or above 10. Illustrative non-limitative examples of "alkaline solutions" include carbonates, hydroxides, phosphates, hydrogen carbonates, phenolates or hydrogen phosphates, among others. In one embodiment, optionally in combination with any of the embodiments provided above or below, the alkaline aqueous solution is a hydroxide aqueous solution.

In one embodiment, optionally in combination with any of the embodiments provided above or below, the solubilization is performed with an alkaline aqueous solution already comprising the cations intended to form the desired tungstate salt. Thus, in one embodiment, optionally in combination with any of the embodiments provided above or below, the alkaline solution comprises cations selected from alkaline or alkaline earth metal cations such as: sodium, potassium, magnesium, and calcium; but also of other cations such as ammonium, cupper, zinc and silver. In one embodiment, optionally in combination with any of the embodiments provided above or below, the alkaline aqueous solution comprises sodium cations.

In an alternative embodiment, optionally in combination with any of the embodiments provided above or below, the solubilization is performed with an alkaline aqueous solution comprising cations other than those intended to form the desired tungstate salt. In this embodiment, a further solution of the intended cation would be added to the already formed tungstate salt. For example, a sodium salt/MeOH, thus obtaining the desired tungstate salt.

In one embodiment, optionally in combination with any of the embodiments provided above or below, the tungsten (VI) salt hydrate is $Na_2WO_4 \cdot 2H_2O$, and the alkaline aqueous solution is a sodium hydroxide aqueous solution.

When performing the solubilization, the solution can be heated at a temperature lower than the reflux temperature in order to facilitate or speed-up the solubilization of the tungsten-containing compound. In one embodiment, optionally in combination with any of the embodiments provided above or below, step (a) is performed at a temperature from 30°C to reflux temperature.

In one embodiment, optionally in combination with any of the embodiments provided above or below, step (a) is performed by solubilizing $WO_3$ in a NaOH aqueous solution; particularly at a temperature from 80 to 95°C; particularly at 85-95°C; particularly at 85, 86, 87, 88, 89, 90, 91, 92, 93, 94 or 95°C. In this embodiment the NaOH is in a molar excess vs the tungsten-containing compound.

In an alternative embodiment, optionally in combination with any of the embodiments provided above or below, step (a) is performed by solubilizing a paratungstate salt, such as the ammonium paratungstate salt (APT) in a NaOH aqueous solution, particularly at a temperature from 50 to 70°C, particularly at 55, 56, 57, 58, 59 or 60°C. In this embodiment the NaOH is in a molar excess vs the tungsten-containing compound.

In an alternative embodiment, optionally in combination with any of the embodiments provided above or below, step (a) is performed by solubilizing $H_2WO_4$ in a NaOH aqueous solution, particularly at a temperature from 30 to 50°C, particularly at 30, 31, 32, 33, 34, 35, 36, 37, 38, 39, 40, 41, 42, 43, 44, 45, 46, 47, 48, 49 or 50°C. In this embodiment the NaOH is in a molar excess vs the tungsten-containing compound.

Once step (a) has been completed and the tungsten-containing compound has been solubilized in the alkaline aqueous solution, the resulting solution is filtered in order to eliminate the impurities. To that end, the filtration is performed at a temperature which guarantees the tungstate salt already formed in step (a) remaining solubilized.

The skilled person can, following their general knowledge, optimize the T for the filtration on the basis of, for example, how concentrate is the tungstate salt solution resulting from step (a). If it is highly concentrated solution (close to salt saturation, for instance), the temperature required will be higher than the one required if the solution is at low concentrations. The optimization of the appropriate T can also be easily determined: those guaranteeing the tungstate salt to be dissolved. If the skilled person detected the undesired precipitation in the solution, this would mean that the filtration should be performed at a higher T.

In one embodiment of the invention, the filtration of step (b) is performed at a temperature equal or higher than 30°C and lower than the reflux temperature.

The filtration can be performed using any of the well-known techniques and commercially available filters. Illustrative non-limitative examples are filters, precoat filters, Cuno filters, sintered glass filters, among others. In the case of precoat filters, they are layers of a filtration medium such as cellulose, perlite, diatomaceous earth that are deposited on the surface of a filter element inside an auto-Jet pressure leaf filter, for instance, following manufacturer's instructions. During filtration, the precoat layer is the one responsible for retaining particles as small as half a micron. Celite is one of the commercially available materials for preparing these precoat filters, but other suitable materials such as sand or activated carbon, for instance, can also be used in the context of the invention.

Once the filtration step has been completed, the filtrate is subjected to the precipitation step.

The precipitation step can be performed by any suitable technique, such as by adding a counter solvent, concentration (evaporating the water to oversaturate the solution), cooling at a temperature below the saturation point (to reduce the solubility of the salt), adding a highly soluble salt containing the same metal as the one already forming the tungstate salt (inducing the precipitation by common ion effect), or a combination thereof.

In one embodiment, optionally in combination with any of the embodiments provided above or below, the precipitation step is performed by cooling at a temperature below the saturation point and/or by adding a water miscible solvent.

In one embodiment, optionally in combination with any of the embodiments provided above or below, the precipitation step is performed by adding a water-miscible solvent having a boiling temperature below 100°C.

In the context of the invention, "water-miscible solvent" refers to a solvent that has a boiling temperature below 100°C and it is miscible with water resulting in homogeneous solutions in a range of solvent/water ratios from 1:100 up to 100: 1 (v/v), from 1 :20 up to 20:1 (v/v), from 1 :2 to 2:1 (v/v) at a temperature of 25°C.The water-miscible solvent is not particularly restricted, but it is preferred that the water-miscible solvent is pharmaceutically/dietetically/veterinarily acceptable. The solvent can be a protic or an aprotic solvent. Suitable protic solvents comprise primary, secondary and tertiary alcohols with a straight or branched, cyclic or acyclic alkyl chain with one to six carbon atoms, carbon acids, primary or secondary amines with a straight or branched, cyclic or acyclic alkyl chain with one to six carbon atoms. Preferred protic solvents comprise primary, secondary and tertiary alcohols with a straight or branched acyclic alkyl chain with one to eight carbon atoms, particularly with one to 6 carbon atoms, such as methanol, ethanol, propan-2-ol; acetic acid or propylamine.

In the context of the invention, the term "water-miscible solvent comprises" embraces those solvents incorporating some content of water. Thus, a water-miscible solvent comprising an alkanol, will embrace any solvent comprising the alkanol+water, too.

In another embodiment, optionally in combination with any of the embodiments provided above or below, the water-miscible solvent comprises or consists of a $(C_1-C_8)$alkanol, ketones, nitriles, sulfoxides, amines, amines, or a mixture thereof. In another embodiment, optionally in combination with any of the embodiments provided above or below, the water-miscible solvent comprises, in addition, water. In one embodiment, optionally in combination with any of the embodiments provided above or below, the crystallization is performed by adding a mixture of water+$(C_1-C_8)$alkanol, particularly of a mixture of water+$(C_1-C_8)$alkanol. In an alternative embodiment, optionally in combination with any of the embodiments provided above or below, the crystallization step is performed by adding a water-miscible solvent consisting of a $(C_1-Cs)$alkanol, particularly a $(C_1-C_4)$alkanol, such as methanol, ethanol, or isopropanol, among others.

Once the precipitation step has been completed, the solid is filtered. Illustrative non-limitative examples of filtration are centrifuge or Nutsche Filte, following manufacturer's instructions.

Once the filtration has been completed the resulting cake is washed with a water-miscible solvent. This step is essential because the aim is to reduce the amount of water wetting the solid, thus minimizing the requirements of the subsequent drying step. To that end, the wet cake is washed with a water-miscible solvent such as those suitable in step (c) for the precipitation of the salt. The washing step can be repeated as many times as considered appropriate to minimize the amount of wetting water: the lower the wetting water, the softer the drying temperature. The several washes can be performed using the same water-miscible solvent or using a different solvent each time.

In one embodiment, optionally in combination with any of the embodiments provided above or below, the washing is performed in two sub-steps: a first one with a solvent comprising water+$(C_1-C_8)$alkanol, and a second one only with a $(C_1-C_8)$alkanol, particularly a $(C_1-C_4)$alkanol, to make the cake expelling as much water as possible, soaking it in this volatile alcohol.

Once the washing step has been completed, it is performed the drying step. This step is performed under conditions that allow the removal of the water-miscible solvent. The skilled person in the art can routinely determine the most appropriate conditions of temperature, time or pressure, among others, to achieve the removal of the solvent. The expression "removal" has to be understood as the elimination of the solvent at a level (residue) which makes the salt acceptable for human or veterinary or dietary use. It forms part of the general knowledge of the skilled person in the art to know the appropriate levels of the solvent in the end product. And the skilled person has access to guidelines on support, such as the ICH Q3C (R8) Residual solvents available on EMEA web-page. Thanks to the washing step, the drying step can be efficiently performed at room temperature in a very short period of time (as shown below). Vacuum can be used to speed-up the drying step. In the examples provided below, the product was efficiently dried in 1 hour under vacuum. Alternatively to vacuum, other illustrative-non limitative suitable techniques is counter air flow with stirring. Depending on the batch scale and on the equipment used to dry the product, the time required will be one or another. For instance, drying a large batch in a drying oven would take much longer than drying a small batch in a Nutcha filter equipped with stirring and heating devices. But in any case the temperature at which the drying is performed is so mild that the hydrate molecules are retained in the final product.

In one embodiment, optionally in combination with any of the embodiments provided above or below, the process is performed at a temperature below 50°C, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C.

In one embodiment, optionally in combination with any of the embodiments provided above or below, the invention provides a process for obtaining $Na_2WO_4 \cdot 2H_2O$, the process comprising:

(i) Preparing the tungsten (VI) salt in an alkaline aqueous solution by dissolving the tungsten-containing compound selected from $WO_3$, a paratungstate salt or tungstic acid in a sodium alkaline aqueous solution at a temperature from 30 to 95°C;

(ii) Filtering the aqueous solution at a temperature from 30 to 60°C;

(iii) Precipitating the dihydrate tungsten (VI) salt from the filtrate resulting from step (b1) by adding a water/miscible solvent comprising or consisting of $(C_1-C_8)$alkanol;

(iv) Filtering;

(v) Washing the resulting filter cake with a water-miscible solvent comprising or consisting of $(C_1-C_8)$alkanol; and

(vi) Drying at a temperature below 50, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C; particularly from 18 to 22°C.

In another embodiment, optionally in combination with any of the embodiments provided above or below, the present invention provides a process for the obtaining of $Na_2WO_4 \cdot 2H_2O$ which comprises:

(a1) Preparing the tungsten (VI) salt in an alkaline aqueous solution by dissolving $WO_3$ in a sodium alkaline aqueous solution at a temperature from 80 to 95°C;

(b1) Filtering the aqueous solution at a temperature from 50 to 100°C, particularly from 50 to 90°C;

(c1) Precipitating the dihydrate tungsten (VI) salt from the filtrate resulting from step (b1) by adding a water/miscible solvent comprising or consisting of $(C_1-C_8)$alkanol;

(d1) Filtering;

(e1) Washing the resulting filter cake with a water-miscible solvent comprising or consisting of $(C_1-C_8)$alkanol; and

(f1) Drying at a temperature below 50, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C; particularly from 18 to 22°C.

In another embodiment, optionally in combination with any of the embodiments provided above or below, the present invention provides a process for the obtaining of $Na_2WO_4 \cdot 2H_2O$ which comprises:

(a2) Preparing the tungsten (VI) salt in an alkaline aqueous solution by dissolving APT in a sodium alkaline aqueous solution at a temperature from 50 to 70°C;

(b2) Filtering the aqueous solution at a temperature from 35 to 60°C, particularly from 40 to 50°C, particularly 45°C;

(c2) Precipitating the dihydrate tungsten (VI) salt from the filtrate resulting from step (b2) by adding a water-miscible solvent comprising or consisting of $(C_1-C_8)$alkanol;

(d2) Filtering;

(e2) Washing the resulting filter cake with a water-miscible solvent comprising or consisting of $(C_1-C_8)$alkanol; and

(f2) Drying at a temperature below 50, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C; particularly from 18 to 22°C.

In another embodiment, optionally in combination with any of the embodiments provided above or below, the present invention provides a process for the obtaining of $Na_2WO_4 \cdot 2H_2O$ which comprises:

(a3) Preparing the tungsten (VI) salt in an alkaline aqueous solution by dissolving $H_2WO_4$ in a sodium alkaline aqueous solution at a temperature below 40°C;

(b3) Filtering the aqueous solution at a temperature from 30 to 60°C, particularly from 35 to 45°C, particularly 35°C;

(c3) Precipitating the dihydrate tungsten (VI) salt from the filtrate resulting from step (b3) by adding a water-miscible solvent comprising or consisting of $(C_1-C_8)$alkanol;

(d3) Filtering;

(e3) Washing the resulting filter cake with a water-miscible solvent comprising or consisting of $(C_1-C_8)$alkanol; and

(f3) Drying at a temperature below 50, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C; particularly from 18 to 22°C.

Advantageously, as it has been widely discussed along the document, the process of the invention allows the obtaining of a tungsten salt with a reduced amount of impurities as well as of residual solvents, making these salts suitable in the fields of pharmacy, veterinary and dietary.

As it is shown below, and by the way of illustration, the resulting sodium tungstate dihydrate, obtained following the process of the invention, had a residual content in MeOH below 3000 ppm, which is the level recognized as safe by the ICH Q3C (R8)- Residual solvents available on EMEA web-page (EMA/CHMP/ICH/82260/2006):

| Solvent | PDE (mg/day) | Concentration limit (ppm) |
| --- | --- | --- |
| Acetonitrile | 4.1 | 410 |
| Chlorobenzene | 3.6 | 360 |
| Chloroform | 0.6 | 60 |
| Cumene[1] | 0.7 | 70 |
| Cyclohexane | 38.8 | 3880 |
| Cyclopentyl methyl ether[2] | 15.0 | 1500 |
| 1,2-Dichloroethene | 18.7 | 1870 |
| Dichloromethane | 6.0 | 600 |
| 1,2-Dimethoxyethane | 1.0 | 100 |
| N,N-Dimethylacetamide | 10.9 | 1090 |
| N,N-Dimethylformamide | 8.8 | 880 |
| 1,4-Dioxane | 3.8 | 380 |
| 2-Ethoxyethanol | 1.6 | 160 |
| Ethyleneglycol | 6.2 | 620 |
| Formamide | 2.2 | 220 |
| Hexane | 2.9 | 290 |

(continued)

| Solvent | PDE (mg/day) | Concentration limit (ppm) |
|---------|--------------|---------------------------|
| Methanol | 30.0 | 3000 |

In a further aspect the present invention provides pharmaceutical, veterinary and food compositions as well as therapeutic uses based on the novel sodium tungstate salt of the invention.

In one embodiment of the invention, the subject treated is a human. In an embodiment, the tungsten (VI) salt of the present invention is useful for the preparation of a medicinal product for the treatment of female infertility occurring with a change in the hypothalamic-pituitary axis. The diseases or conditions occurring with a change in the hypothalamic-pituitary axis include, among others, polycystic ovarian syndrome, metabolic syndrome, hyperprolactinemia, endometriosis, eating disorders, obesity, hypothyroidism, multiple sclerosis, rheumatoid arthritis, lupus erythematosus, cirrhosis, celiac disease, chronic renal failure and idiopathic causes.

In another embodiment, the tungsten (VI) salt of the present invention is useful for the preparation of a medicinal product for the treatment of female idiopathic infertility. The concepts "idiopathic infertility" or "infertility without an apparent cause" or "unexplained infertility" have the same meaning and are used interchangeably. These concepts refer to those infertility cases in which the reason for the inability to achieve pregnancy has not been found in the standard sterility tests.

In another embodiment, the tungsten (VI) salt of the present invention is useful for the preparation of a medicinal product for the treatment of female infertility due to eating disorders. The eating disorders occurring with a change in the hypothalamic-pituitary axis include, among others, anorexia nervosa and bulimia.

In another embodiment, the treatment of infertility comprises restoring the ovulation. The term "ovulation" is understood as that process through which a woman releases oocytes into the uterus, and the term "restoring ovulation" is understood as the process through which the menstrual cycle is reestablished, i.e., it is re-established the process of regularly releasing the oocyte into the uterus in women that did not ovulate (anovulation) or ovulated irregularly (oligoovulation).

In another embodiment, the treatment of infertility comprises increasing the number of oocytes implanted into the uterus wall or for improving the chance of successful pregnancy without increasing the risk of multiple pregnancies. The term "increasing zygote implantation" is understood as the process through which the efficiency of the implantation of the oocytes into the uterus is increased.

One embodiment of the invention, the pharmaceutical, veterinary or food composition is a composition for oral administration. Solid oral compositions such as tablets and capsules, and liquid oral compositions such as oral solutions or suspensions, are of special interest.

The expression "pharmaceutically or veterinarily or dietary acceptable excipient or carrier" refers to the excipients or carriers suitable for use in pharmaceutical, veterinary or dietary technology for the preparation of the compositions for medical use. These components, excipients and carriers, must be compatible with the other ingredients of the composition. It must also be suitable for use in contact with human or animal tissues or organs without excessive toxicity, irritation, allergic response, or other immunogenicity problems or complications with a reasonable risk/benefit ratio.

As used herein, the expression "therapeutically effective amount" refers to the amount of the tungsten (VI) salt which, when administered, is sufficient for the treatment of female infertility defined in the present invention. The specific doses of the tungsten (VI) salt according to this invention is determined by the particular circumstances surrounding the case, including, for example, the administered salt, the administration route, the administered pharmaceutical, veterinary or food composition, as well as the characteristics of the patient including, among others, height, weight and age; and the nature and stage of the disease. For example, an amount of a tungsten (VI) salt defined above comprised from 50 to 500 mg/kg/day can be used.

The following examples are given in order to provide a person skilled in the art with a sufficiently clear and complete explanation of the present invention, but should not be considered as limiting of the essential aspects of its subject, as set out in the preceding portions of this description.

**Examples**

Example 1: Synthesis and isolation of $Na_2WO_4 \cdot 2H_2O$ starting from APT

2.0 equivalents of NaOH(s) were added to APT suspensions containing 1.52 volumes of water.

This resulted in suspensions of the same starting products in water, which only evolved when the mixtures were heated to 55 °C in the case of the APT experiments. The need to heat the initial mixtures is attributed to the low water solubility of APT.

Once the sodium tungstate solutions were obtained, they were filtered with Celite precoat to remove insoluble impurities. These filtrations were carried out hot (45°C) to avoid retaining part of the desired product, since in order to obtain a higher recovery, the salt concentration in the crude is close to its saturation point at room temperature.

After filtering the solution, a proportion of MeOH equivalent to 1.27 times the amount of water in the filtrate was added. This resulted in fluid white suspensions, which could be filtered through sintered glass funnels, without the need for recirculation.

In addition to washing the solid with the same MeOH/$H_2O$ ratio as the reaction crude (14:11), a final wash with MeOH was carried out so that the solid expels as much water as possible and is soaked in this volatile alcohol. Once well drained, airflow was applied by applying a vacuum to the plate itself for 1 hour.

To determine whether entrainment with MeOH or entrainment with air also led to the undesired dehydration of the $Na_2WO_4 \cdot 2H_2O$ salt, 5.6131 g of each of the solids obtained in two analogous batches were dried for 15 hours in a vacuum oven at 105 °C.

The weight loss corresponded approximately to the weight of two waters, for which a final weight of 5.000 g would be expected.

The weights obtained were 5.0092 and 5.0099 g for the solids of two analogous batches.

The results showed, on the one hand, that the drying method conditions of the invention are efficient and, on the other hand, that the initial solid obtained is a dihydrate. The presence of water molecules in the room temperature dried solids was also evidenced by recording their IR spectra, ("Spectrum Two" de Perkin Elmer, 120100) which show the same bands as the standard compound $Na_2WO_4 \cdot 2H_2O$ (Fig. 1).

In terms of yields, the APT-based experiments reached 81%.

Example 2: Synthesis and isolation of $Na_2WO_4 \cdot 2H_2O$ starting from $WO_3$

The same procedure as the one of Example 1 was followed starting from a $WO_3$ suspension in 3.07 volumes of $H_2O$, but with the following minor changes in the solubilization step: 4.0 equivalents of NaOH were added in the case of $WO_3$ suspensions, and the resulting suspensions were heated to 90 °C.

Again, it was determined the dehydration of the $Na_2WO_4 \cdot 2H_2O$ salt. To do so, 5.6131 g of each of the solids obtained in two analogous batches were dried for 15 hours in a vacuum oven at 105 °C.

The weight loss corresponded approximately to the weight of two waters, for which a final weight of 5.000 g would be expected.

The weights obtained were 5.0086 and 5.0103 g for the solids of two analogous batches.

The results showed, again, that the drying method conditions of the invention are efficient and, on the other hand, that the initial solid obtained is a dihydrate. The presence of water molecules in the non-temperature dried solids was also evidenced by recording their IR spectra, which show the same bands as the standard compound $Na_2WO_4 \cdot 2H_2O$ (Fig. 1).

In terms of yields, it was achieved a 95% and 96% for two performed $WO_3$-based experiments.

The higher performance of the experiments starting from $WO_3$ is attributed to the fact that the higher salt load of their respective crudes, caused by the need to add twice as many NaOH equivalents to generate the sodium salt, allows the precipitation of a higher percentage of the salt than in the crudes starting from APT.

Example 3: Synthesis and isolation of $Na_2WO_4 \cdot 2H_2O$ starting from $H_2WO_4$

The same process as the one provided in Example 1 was followed but with some minor modifications.

The solubilization step was performed by slowly adding 2.0 equivalents of NaOH to a cold suspension of the acid in 2.1 volumes of water. In order to achieve a higher recovery, the initial amount of water was adjusted to the amount needed to dissolve the salt obtained at r.t.

After heating the resulting solution at 40 °C, it was filtered with Celite to retain the insoluble impurities, obtaining the $Na_2WO_4$(aq) solution from which the final sodium tungstate was isolated by precipitation under the same conditions as those pointed out in Example 1.

In this case, the precipitation was performed by adding 2.0 volumes of either MeOH, EtOH, acetone or IPA at room temperature. After overnight stirring at room temperature, the suspensions were filtered at room temperature, and the cakes were washed by percolation with 0.4 volumes of $H_2O$/solvent (1:1), and with 0.4 volumes of the corresponding solvent. The final wet cakes were dried overnight at 100°C.

Achieved chemical yield using each solvent was 85% in the case of MeOH, 80% in the case of EtOH, 50% IPA, 69% Acetone. Although the yield using EtOH is close to that of MeOH, the latter was chosen because of its lower cost, its affordability, and because the lower boiling point should facilitate the drying of the final product.

This point was shown to be of utmost importance because $Na_2WO_4 \cdot 2H_2O$ waters of hydration were found to be extremely labile. In fact, in the different drying processes at 100-110 °C of these and other synthesized solids, dehydration processes of the tungsten salt were detected. This phenomenon was observed when recording the IR spectra of the synthesized and dried products, as none of them show the stretching and bending bands typical of water molecules (Fig. 2).

The same process was repeated but drying the final cake at room temperature (r.t.) and consequently preventing the loss of the water of crystallization of the final product. The products were dried at room temperature, in an open container without forced airflow. The weight after two days at room temperature is the same as after 4 days under the same conditions. It should be determined whether the process can be accelerated by forcing air through the solid, and what is the maximum temperature that will not cause dehydration of the solid.

The synthesis yields of the final acid-product stage were 70-72%.

Example 4: analytical test

The two direct synthesis scale-ups of $Na_2WO_4 \cdot 2H_2O$ from $WO_3$ were analysed by ICP-MS (ICP-MS VARIAN 820) as they are the ones that, based on previous analyses, are postulated as the candidates with the highest purity.

As far as metallic impurities are concerned, both products comply with the specifications. The sodium and tungsten content were also analysed (Table 1).

| Table 1. ICP-MS results of $Na_2WO_4 \cdot 2H_2O$ synthesised directly from $WO_3$* | | | |
|---|---|---|---|
| | Specification | Batch 1 | Batch 2 |
| Metals (ICP-MS) | | | |
| Lead (Pb) | NMT 5.0ppm | Compliant (<0.5 ppm) | Compliant (<0.5 ppm) |
| Cadmium (Cd) | NMT 5.0ppm | Compliant (<0.05 ppm) | Compliant (<0.05 ppm) |
| Arsenic (As) | NMT 5.0ppm | Compliant (<1 ppm) | Compliant (<1 ppm) |
| Vanadium (V) | NMT 20.0ppm | Compliant (<10 ppm) | Compliant (<10 ppm) |
| Nickel (Ni) | NMT 20.0ppm | Compliant (<1 ppm) | Compliant (<1 ppm) |
| Cobalt (Co) | NMT 20.0ppm | Compliant (<10 ppm) | Compliant (<10 ppm) |
| Mercury (Hg) | NMT 0.1ppm | Compliant (<0.1 ppm) | Compliant (<0.1 ppm) |
| | | | |
| Content | | | |
| %Tungsten (W) | 59.5-65.7** | 60.1±14.4 | 63.8±14.7 |
| % Sodium (Na) | 14.9-16.5** | 17.0±3.9 | 13.6±3.1 |
| *Expressed on dry matter. ** 95-105% of the theoretical. | | | |

It was also determined the MeOH amount in the end product following the protocol provided below:
i. Preparation of the solutions:

Blank solvent: Introduce 250 mg of sodium chloride and 1.0 mL of water into a headspace vial of 10 mL.

Standard solution: Prepare in duplicate. Weigh accurately 150 mg of methanol standard into a 10.0 mL volumetric flask with 5 mL of water. Dissolve and make up to volume with water.

Homogenize the solution. Add 1.0 mL of the previous solution into a 50.0 mL volumetric flask. Make up to volume with water, and homogenize the solution. Introduce 250 mg of sodium chloride and 1.0 mL of the previous standard solution into a headspace vial of 10 mL.

Test solution: Prepare in duplicate. Introduce 250 mg of sodium chloride, 100 mg of the sample and 1.0 mL of water into a headspace vial of 10 mL.

ii. Chromatographic conditions:
Equipment: Gas chromatograph equipped with FID detector and headspace sampler (HSS).

Column: Agilent BPX-VOL, 30 m x 0.25 mm ID, 1.4 $\mu$m (or equivalent).
GC Parameters:
Injector: Temperature: 180 °C
Oven temperature gradient:

**Table 2**

| Initial temperature (°C) | Gradient temperature (°C/min) | Final temperature (°C) | Hold time (min) |
|---|---|---|---|
| 40.0 | --- | 40.0 | 0.0 |
| 40.0 | 5.0 | 60.0 | 0.0 |
| 60.0 | 40.0 | 240.0 | 1.5 |

Carrier Gas: He, constant flow mode 1.4 mL/min
Mode Split, Split ratio 25, Split flow 35 mL/min
Detector: Temperature: 270 °C
H2 Flow: 35 mL/min
Air Flow: 350 mL/min
Make up gas Flow (N2): 40 mL/min
Analysis time: 10.0 minutes
Post run: 1.0 minutes

HS Parameters:

Oven temperature: 80°C
Loop temperature: 90°C
Transfer line temperature: 100°C
Vial equilibration: 40 minutes
Injection duration: 0.20 minutes
GC Cycle Time: 20 minutes

Approximate Retention Time (RT) of Methanol: 1.74 minutes
*Acceptance criteria:*

1. Standard solution A:

- Coefficient of variation of the response factor (n=6) should be d 10.0%

It can be concluded that, for the time being, the products obtained by direct reaction from $WO_3$ yield sodium tungstate dihydrate, which comply with the impurity specifications.
2. Standard solution B:

- Ratio of the response factor for STDA-STDB(initial) should be within the 90 - 110%
- Ratio of the response factor for STDB(initial)-STDB(final) should be within the 90 - 110%.

*Results*: the area of the MeOH peak in the test solution was not more than to the area of the MeOH peak in the standard solution, the test complies with the specifications (d 3000 ppm).

Example 5: stability test

The present inventors confirmed the stability of the hydrate resulting from the process of the invention determining the loss on drying with time.
To that end a well-mixed sample of the sodium tungstate hydrate resulting from Example 2, was subjected to the following protocol. Dry clean glass-stopper, shallow weighing bottle under the same conditions to be employed in the determination. Cool to room temperature in a desiccator and take the weight accurately (W1) g. Transfer to the bottle 1 g of the sample, cover it and accurately weigh the bottle and the contents (W2) g. Place the loaded bottle in the drying chamber (oven), remove the stopper and leave it also in the chamber. Dry the sample to the specified time and temperature to a constant mass (105°C). After drying is completed, open the drying chamber, close the bottle promptly and allow it to cool to room temperature (where applicable) in a desiccator before weighing. Weigh the bottle and the contents (W3) g. Calculation:

$$\% \text{ of Loss on drying} = \frac{(W_2 - W_3) \times 100}{(W_2 - W_1)}$$

The loss on drying should be not more than 15 % (w/w).

It was found that the hydrate resulting from the process of the invention showed a loss on drying below 15%. Not only that, but also, that the loss was maintained along the 18-month test at 25°C/60%HR and 6-month 40°C/75%HR.

**Claims**

1. A process for obtaining a tungsten (VI) salt hydrate, the process comprising:

   (a) Preparing a tungsten (VI) salt in an alkaline aqueous solution;
   (b) Filtering the aqueous solution at a temperature at which the tungsten (VI) salt remains dissolved;
   (c) Precipitating the tungsten (VI) salt hydrate from the filtrate resulting from step (b);
   (d) Filtering;
   (e) Washing the resulting filter cake with a water-miscible solvent; and
   (f) Drying the washed cake under conditions that allow the removal of the water-miscible solvent.

2. The process of claim 1, wherein the tungsten (VI) salt hydrate comprises an anion selected from the group consisting of tungstate and isopolytungstate, particularly the anion is tungstate.

3. The process according to any one of the preceding claims, wherein the tungsten (VI) salt hydrate comprises a cationic moiety selected from the group of cations: sodium, ammonium, potassium, magnesium, and calcium, zinc, cupper, and silver.

4. The process of any one of the preceding claims, wherein the tungsten (VI) salt hydrate is dihydrate sodium tungstate.

5. The process of any one of the preceding claims, wherein step (a) is performed by dissolving a tungsten-containing compound selected from: $WO_3$, a paratungstate salt, and tungstic acid, in an alkaline aqueous solution.

6. The process of any one of the preceding claims, wherein the alkaline aqueous solution further includes cationic moieties selected from sodium, ammonium, cupper, potassium, magnesium, and calcium, zinc and silver.

7. The process of any one of the preceding claims 5-6, wherein the alkaline aqueous solution is added in a molar excess with respect to the tungsten-containing compound.

8. The process of any one of the preceding claims, wherein step (a) is performed by heating to a temperature lower than the reflux temperature.

9. The process of any one of the preceding claims, wherein the filtering step (b) is performed at a temperature equal or above 30°C but below the reflux temperature.

10. The process of any one of the preceding claims, wherein step (c) is performed by cooling or adding a water-miscible solvent.

11. The process of any one of the preceding claims, wherein the water-miscible solvent used in steps (c) and (e) comprises or consists $(C_1\text{-}C_8)$alkanol.

12. The process of any one of the preceding claims, wherein step (e) is repeated.

13. The process of any one of the preceding claims for obtaining $Na_2WO_4 \cdot 2H_2O$, the process comprising:

(i) Preparing the tungsten (VI) salt in an alkaline aqueous solution by dissolving the tungsten-containing compound selected from $WO_3$, a paratungstate salt or tungstic acid in a sodium alkaline aqueous solution at a temperature from 30 to 95°C;

(ii) Filtering the aqueous solution at a temperature from 30 to 60°C;

(iii) Precipitating the dihydrate tungsten (VI) salt from the filtrate resulting from step (b1) by adding a water/miscible solvent comprising or consisting of $(C_1-C_8)$alkanol;

(iv) Filtering;

(v) Washing the resulting filter cake with a water-miscible solvent comprising or consisting of $(C_1-C_8)$alkanol; and

(vi) Drying at a temperature below 50°C, particularly from 15 to 25°C, particularly from 18 to 22°C; or, alternatively, the process comprising:

(a1) Preparing the tungsten (VI) salt in an alkaline aqueous solution by dissolving $WO_3$ in a sodium alkaline aqueous solution at a temperature from 80 to 95°C;

(b1) Filtering the aqueous solution at a temperature from 50 to 90°C;

(c1) Precipitating the dihydrate tungsten (VI) salt from the filtrate resulting from step (b1) by adding a water/miscible solvent comprising or consisting of $(C_1-C_8)$alkanol;

(d1) Filtering;

(e1) Washing the resulting filter cake with a water-miscible solvent comprising or consisting of $(C_1-C_8)$alkanol; and

(f1) Drying at a temperature from 18 to 22°C; or, alternatively,

the process comprising:

(a2) Preparing the tungsten (VI) salt in an alkaline aqueous solution by dissolving APT in a sodium alkaline aqueous solution at a temperature from 50 to 70°C;

(b2) Filtering the aqueous solution at a temperature from 35 to 60°C;

(c2) Precipitating the dihydrate tungsten (VI) salt from the filtrate resulting from step (b2) by adding a water-miscible solvent comprising or consisting of $(C_1-C_8)$alkanol;

(d2) Filtering;

(e2) Washing the resulting filter cake with a water-miscible solvent comprising or consisting of $(C_1-C_8)$alkanol; and

(f2) Drying at a temperature from 18 to 22°C; or, alternatively,

the process comprising:

(a3) Preparing the tungsten (VI) salt in an alkaline aqueous solution by dissolving $H_2WO_4$ in a sodium alkaline aqueous solution at a temperature below 40°C;

(b3) Filtering the aqueous solution at a temperature from 30 to 60°C, particularly from 35 to 45°C, particularly 35°C;

(c3) Precipitating the dihydrate tungsten (VI) salt from the filtrate resulting from step (b3) by adding a water-miscible solvent comprising or consisting of $(C_1-C_8)$alkanol;

(d3) Filtering;

(e3) Washing the resulting filter cake with a water-miscible solvent comprising or consisting of $(C_1-C_8)$alkanol; and

(f3) Drying at a temperature below 50, 49, 48, 47, 46, 45, 44, 43, 42, 41, 40, 39, 38, 37, 36, 35, 34, 33, 32, 31, 30, 29, 28, 27, 26, 25, 24, 23, 22, 21 or 20 °C; particularly from 18 to 22°C.

**14.** A pharmaceutical, veterinary or dietary sodium tungstate dihydrate comprising a water residual content of up to 15% by weight with respect to the total weight of the salt, particularly a content from 10 to 15% by weight.

**15.** The sodium tungstate salt which is obtainable by the process as defined in anyone of the claims 1-13.

**16.** A pharmaceutical or veterinary or food composition comprising a therapeutically effective amount of the sodium tungstate dihydrate as defined in any of the claims 14-15, together with one or more pharmaceutically or veterinarily or dietary acceptable excipients or carriers.

**17.** A sodium tungstate dihydrate as defined in any of the claims 14-15 for use in therapy.

18. A sodium tungstate dihydrate as defined in any of the claims 14-15 for use in the treatment of female infertility.

**Fig. 1**

**Fig. 2**

Europäisches
Patentamt

European
Patent Office

Office européen
des brevets

# EUROPEAN SEARCH REPORT

Application Number

EP 22 38 3073

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 052 477 A (TAN QIN) 21 December 2018 (2018-12-21) | 1-15 | INV. C01G41/00 A61K33/00 |
| A | * claims * | 16-18 | |
| X | CN 107 572 588 A (DING MING) 12 January 2018 (2018-01-12) | 14-17 | |
| A | * claims * | 1-13,18 | |
| A | PINHEIRO THAÍS N. ET AL: "Sodium Tungstate Dihydrate (Na2WO4.2H2O): A Mild Oxidizing and Efficient Reagent in Organic Synthesis", SYNOPEN, vol. 06, no. 03, 1 September 2022 (2022-09-01), pages 208-210, XP093044219, DOI: 10.1055/a-1924-8008 Retrieved from the Internet: URL:https://www.thieme-connect.com/product s/ejournals/pdf/10.1055/a-1924-8008.pdf> * the whole document * | 1-18 | |
| X | WO 2016/012632 A1 (OXOLIFE SL [ES]) 28 January 2016 (2016-01-28) | 14-18 | TECHNICAL FIELDS SEARCHED (IPC) C01G A61K |
| A | * claims * | 1-13 | |
| X | US 9 675 638 B2 (OXOLIFE SL [ES]) 13 June 2017 (2017-06-13) | 14-18 | |
| A | * claims * | 1-13 | |
| A | US 7 416 894 B2 (MERCK PATENT GMBH [DE]) 26 August 2008 (2008-08-26) * column 1, line 33 – line 35 * | 1-18 | |

The present search report has been drawn up for all claims

| Place of search | Date of completion of the search | Examiner |
|---|---|---|
| Munich | 5 May 2023 | Nobis, Barbara |

EPO FORM 1503 03.82 (P04C01)

EP 4 368 577 A1

## ANNEX TO THE EUROPEAN SEARCH REPORT
## ON EUROPEAN PATENT APPLICATION NO.

EP 22 38 3073

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2023

| Patent document cited in search report | | Publication date | Patent family member(s) | | Publication date |
|---|---|---|---|---|---|
| CN 109052477 | A | 21-12-2018 | NONE | | |
| CN 107572588 | A | 12-01-2018 | NONE | | |
| WO 2016012632 | A1 | 28-01-2016 | AR | 101252 A1 | 07-12-2016 |
| | | | AU | 2014401750 A1 | 09-02-2017 |
| | | | BR | 112017001287 A2 | 30-01-2018 |
| | | | CA | 2955914 A1 | 28-01-2016 |
| | | | CN | 106715332 A | 24-05-2017 |
| | | | DK | 3173382 T3 | 13-03-2023 |
| | | | EP | 3173382 A1 | 31-05-2017 |
| | | | HR | P20230223 T1 | 14-04-2023 |
| | | | JP | 6534439 B2 | 26-06-2019 |
| | | | JP | 2017523170 A | 17-08-2017 |
| | | | KR | 20170029627 A | 15-03-2017 |
| | | | LT | 3173382 T | 27-03-2023 |
| | | | NZ | 728467 A | 30-04-2021 |
| | | | PT | 3173382 T | 10-03-2023 |
| | | | RS | 64025 B1 | 31-03-2023 |
| | | | SG | 11201700485V A | 27-02-2017 |
| | | | SI | 3173382 T1 | 28-04-2023 |
| | | | TW | 201618796 A | 01-06-2016 |
| | | | US | 2017173074 A1 | 22-06-2017 |
| | | | UY | 36222 A | 29-01-2016 |
| | | | WO | 2016012632 A1 | 28-01-2016 |
| | | | ZA | 201701241 B | 25-05-2022 |
| US 9675638 | B2 | 13-06-2017 | AU | 2014209987 A1 | 21-01-2016 |
| | | | BR | 112015017483 A2 | 11-07-2017 |
| | | | CA | 2898953 A1 | 31-07-2014 |
| | | | CL | 2015002047 A1 | 04-01-2016 |
| | | | CN | 105188721 A | 23-12-2015 |
| | | | CY | 1118287 T1 | 28-06-2017 |
| | | | DK | 2948156 T3 | 02-01-2017 |
| | | | EP | 2948156 A1 | 02-12-2015 |
| | | | ES | 2478790 A1 | 22-07-2014 |
| | | | ES | 2611776 T3 | 10-05-2017 |
| | | | HK | 1217442 A1 | 13-01-2017 |
| | | | HR | P20161611 T1 | 27-01-2017 |
| | | | HU | E031910 T2 | 28-08-2017 |
| | | | JP | 6045721 B2 | 14-12-2016 |
| | | | JP | 2016505057 A | 18-02-2016 |
| | | | KR | 20150130992 A | 24-11-2015 |
| | | | LT | 2948156 T | 27-12-2016 |
| | | | MX | 356851 B | 18-06-2018 |
| | | | NZ | 710583 A | 24-06-2016 |

EPO FORM P0459

For more details about this annex : see Official Journal of the European Patent Office, No. 12/82

18

## ANNEX TO THE EUROPEAN SEARCH REPORT
## ON EUROPEAN PATENT APPLICATION NO.

EP 22 38 3073

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2023

| Patent document cited in search report | | Publication date | Patent family member(s) | | | Publication date |
|---|---|---|---|---|---|---|
| | | | PL | 2948156 | T3 | 31-07-2017 |
| | | | PT | 2948156 | T | 30-11-2016 |
| | | | RU | 2015135578 | A | 02-03-2017 |
| | | | SG | 11201505701W | A | 28-08-2015 |
| | | | SM | T201700018 | B | 08-03-2017 |
| | | | US | 2015359820 | A1 | 17-12-2015 |
| | | | WO | 2014114644 | A1 | 31-07-2014 |
| US 7416894 | B2 | 26-08-2008 | DE | 10336571 | B3 | 27-01-2005 |
| | | | EP | 1658491 | A1 | 24-05-2006 |
| | | | US | 2006234380 | A1 | 19-10-2006 |
| | | | WO | 2005017520 | A1 | 24-02-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016012632 A

**Non-patent literature cited in the description**

- **J. BALLESTER.** Tungstate administration improves the sexual and reproductive function in female rats with streptozotocin-induced diabetes. *Human Reproduction,* 2007, vol. 22, 2128-2135